# Europäisches Patentamt
## European Patent Office
## Office européen des brevets

(19)

(11) Veröffentlichungsnummer: **0 027 278**

**A1**

(12) **EUROPÄISCHE PATENTANMELDUNG**

(21) Anmeldenummer: **80106306.6**

(22) Anmeldetag: **16.10.80**

(51) Int. Cl.³: **C 02 F 1/76**
**C 02 F 1/32**

(30) Priorität: **16.10.79 DE 2941742**

(43) Veröffentlichungstag der Anmeldung:
**22.04.81 Patentblatt 81/16**

(84) Benannte Vertragsstaaten:
**AT BE CH DE FR GB IT LI LU NL SE**

(71) Anmelder: **Schenck, Günther Otto, Prof. Dr.**
**Bismarckstrasse 31**
**D-4330 Mülheim-Ruhr(DE)**

(72) Erfinder: **Schenck, Günther Otto, Prof. Dr.**
**Bismarckstrasse 31**
**D-4330 Mülheim-Ruhr(DE)**

(74) Vertreter: **Wolgast, Rudolf, Dr. et al,**
**Bökenbusch 41 Postfach 11 03 86**
**D-5620 Velbert 11 Langenberg(DE)**

(54) **Kombiniertes Jod-UV-Desinfektionsverfahren für Wasser.**

(57) Zur Desinfektion von Wasser in offenen Systemen wie Trinkwasserversorgungen wird das Wasser mit Jod unter Ausbildung einer Konzentration von 0.2 ppm versetzt und vor oder nach der Jodzugabe in einem Durchflußreaktor mit einer vorbestimmten Mindestdosis von UV-Strahlung im Wellenlängenbereich von 250 bis 320 nm bestrahlt. Zur Desinfektion eines Wasservorrats 2 in einem geschlossenen System wie einem Schwimmbad wird das in dem Schwimmbecken 1 enthaltene Wasser mit Jodid und einem das Jodid zu Jod oxidierenden Oxidationsmittel unter Ausbildung einer von den Anforderungen abhängigen stationären Jodkonzentration versetzt und umgewälzt. In dem Umwälzkreislauf befinden sich eine Filteranlage 11, eine UV-Bestrahlungseinrichtung 12 zur Bestrahlung des umlaufenden Wassers mit einer vorbestimmten Mindestdosis von UV-Strahlung im Wellenlängenbereich von 250 bis 320 nm und eine Zufuhrvorrichtung 13 für Oxidationsmittel zur Reoxidation von Jodid. Die Zufuhrvorrichtung 13 wird entsprechend dem Redoxpotential des Wasservorrats 2 gesteuert dessen pH-Wert auf 7,2-8 eingestellt wird.

BEZEICHNUNG
siehe Titelseite

Die Erfindung betrifft ein Verfahren zur Desinfektion von
Wasser mit elementarem Jod, bei dem das Wasser mit Jod versetzt wird. Sie betrifft auch ein Verfahren zur Desinfektion
eines Wasservorrats, der wenigstens teilweise, gegebenenfalls
unter Zwischenschaltung einer Filteranlage, umgewälzt wird,
mit elementarem Jod, bei dem der Wasservorrat mit Jodid in
der wirksamen Menge, die mindestens zur Erzeugung der zur
Desinfektion notwendigen Konzentration an elementarem Jod
(in mg/l = ppm) ausreicht, und mit einem Oxidationsmittel
in einer Menge versetzt wird, die mindestens zur Erzeugung
einer wirksamen Konzentration an elementarem Jod ausreicht,
und bei welchem Verfahren das Jodid nach Maßgabe seines Verlustes und das Oxidationsmittel nach Maßgabe seines Verbrauchs ergänzt werden, sowie der pH-Wert des Wasservorrats
im Bereich zwischen 6 bis 8 gehalten wird.

Solche Verfahren sind für die Desinfektion von Wasser in offenen Systemen wie Trinkwasserversorgungen, aber auch zur Desinfektion von Wasser in geschlossenen Systemen wie Schwimmbädern oder Kühlwasserkreisläufen geeignet.

Die Desinfektionswirkung des Jods und der im wässrigen System im Gleichgewicht vorliegenden Unterjodigen Säure HOJ steht der der übrigen Halogene (Chlor und Brom) nicht nach, gegenüber Staphylokokkus aureus und Pseudomonas aeruginosa (L.Gershenfeld, B.Witlin, Amer. J. Pharm. 121 (1949), S. 95 bis 104) sowie besonders gegenüber den Zysten der Endamöba histolytica hat sich Jod als überlegen erwiesen (S.L.Chang, J. Amer. pharm. Assoc. 47 (1958), S. 417 bis 423).

Elementares Jod ist bereits im ersten Weltkrieg zur Desinfektion von Trinkwasser von der Französischen Armee eingesetzt worden (G.C.White "Zur Geschichte der Jodierung von Wasser", Handbook of Chlorination, Van Nostrand Reinhold Company, New York 1972, S. 687 bis 697). Ein Langzeitversuch 1948/49, bei dem eine große Zahl von US-Soldaten für sechzehn Wochen im Mittel 12 mg Jod/Tag und für zehn Wochen im Mittel 19.2 mg Jod/Tag aufgenommen hatten, ohne daß irgendwelche nachteilige Folgen beobachtet wurden, führte zur uneingeschränkten Anerkennung der Verwendung von Jod zur Trinkwasserdesinfektion. In der revidierten Auflage von 1962 des "U.S. Public Health Service Manual of Individual Water Supply Systems" ist auch die Jodierung für die Desinfektion von Wasserversorgungen aufgenommen. Eine nochmalige über neunzehn Monate ausgedehnte Überprüfung der Jodierung der Trinkwasserversorgung einer geschlossenen Anstalt mit ca. 700 Personen ergab ebenfalls keinen Hinweis auf nachteilige Folgen für die allgemeine Gesundheit oder auf die Schilddrüsen-Funktion (A.P.Black, R.N.Kinmann, W.C.Thomas jr., G.Freund, E.D.Bird, J. Amer. Waterworks 57 (1965), S. 1401 bis 1421). Der Vorteil des Jods bei der Desinfektion von Trinkwasser wird vor allem darauf zurückgeführt,

daß es infolge seiner im Vergleich zu Chlor wesentlich geringeren Reaktionsfähigkeit nur in relativ geringem Maße mit den
im Wasser vorliegenden Verunreinigungen, insbesondere nicht mit
Ammoniak, reagiert und daher über lange Zeiten sein Desinfektionsvermögen behält. Die Dosierung ist daher auch viel einfacher und weniger kritisch als die des wegen seiner hohen
Reaktionsfähigkeit in mannigfaltigen Nebenreaktionen aufgezehrten Chlors.

Die entsprechenden Beobachtungen sind für die Desinfektion von
Schwimmbadwasser seit 1948 bekannt geworden, wobei beschrieben
wird, daß das elementare Jod im wesentlichen zu Jodid reduziert
wird und hieraus durch Oxidationsmittel wie Chlor oder Chloramin etc. regeneriert werden kann, so daß das Verfahren hier
als ein oxidativ katalytisches Desinfektionsverfahren anzusehen ist. Je nach Reaktionsumständen kann der Regenerationszyklus 10 bis 13-fach wiederholt werden. Dabei ist das erforderliche Chlor bzw. Oxidationsmittel gasförmig oder als Chlorwasser, Hypochloritlösung oder Hypochloritsalz oder in Form
von Chloramin gemäß der beobachteten Jodzehrung so zuzugeben,
daß die Bildung von solchen Verbindungen noch unterbleibt, die
Schleimhaut- und Augenreizungen verursachen können. Das Chlor
oder Chloramin kann dabei im stöchiometrischen Überschuß oder
Unterschuß zugesetzt werden (US-PS 2 443 429; A.P.Black,
R.N.Kinmann, M.A.Keirn, J.J.Smith, W.H.Harlan, Amer. J. Public
Health 60 (1970), S. 535 bis 545).

Trotz einer Reihe besonders günstiger Eigenschaften hat die
Jod-Desinfektion von Wasser wenig Verbreitung gefunden, da das
Verfahren mit verschiedenen Nachteilen verbunden ist, die seinen
Einsatz bisher erschwert, wenn nicht unmöglich gemacht haben.
An erster Stelle steht der im Vergleich zu Chlor um eine Größenordnung höhere Preis des Jods, der sich am stärksten auf die
nicht katalytischen Verfahren auswirkt. In der Entkeimung von
Schwimmbadwasser folgt als ernstester Mangel das Auftreten einer gelbbraunen Färbung des Beckenwassers bei 0.6 ppm Jod und
darüber (G.C.White, l.c., S. 697; E.V.Putnam, Parks & Recreation,
The American Institute of Park Executives, Inc., April 1961),
die die normale Benutzung des Wassers zum Schwimmen und Tauchen
infrage stellt. Ferner kommt es zur Vermehrung von jodresistenten
Keimen (Alcaligenes faecalis und Pseudomonas alcaligenes) vor
allem im Filtersystem, weshalb zu hohe Gesamtkeimzahlen gefunden
werden, die die Beurteilung des sanitären Zustandes des Wassers
durch die Gesamtkeimzahl verfälschen (M.S.Favero, Z.H.Drake,
Appl.Microbiol. 14 (1966), S. 627 bis 653). Schließlich werden
die algiziden Eigenschaften (J.B.Lackey, E.W.Lackey, G.B.Morgan,
Eng.Brog.Univ. FLA., XVIII, Nr. 3 (1964), S. 1 bis 8, Leaflet 171)
des Jods in Gegenwart von Ammoniak neutralisiert (G.C.White, l.c.,
S. 696), weshalb oft von Zeit zu Zeit eine Überchlorung zwecks
Algenkontrolle erforderlich ist.

Der Erfindung liegt die Aufgabe zugrunde, ein Desinfektionsverfahren der vorgenannten Art anzugeben, bei dem bei möglichst
niedrigen Jodkonzentrationen durch Kombination mit einem weiteren Desinfektionsverfahren die Nachteile des bisherigen Verfahrens überwunden werden.

Erfindungsgemäß wird diese Aufgabe dadurch gelöst, daß das
Wasser mit einer wirksamen Dosis von UV-Strahlung im Wellenlängenbereich von 250 bis 320 nm bestrahlt wird.

Erfindungsgemäß wird diese Aufgabe bei einem geschlossenen
System dadurch gelöst, daß das Wasser nach Zugabe der nötigen
Menge an Jodid im Umwälzkreislauf, gegebenenfalls nach Passieren
der Filteranlage, mit einer wirksamen Dosis von UV-Strahlung im
Wellenlängenbereich von 250 bis 320 nm bestrahlt und das Wasser
anschließend mit dem Oxidationsmittel versetzt wird.

Die Erfindung geht aus von der bakteriziden Wirkung der UV-Strahlung, deren Wirkungsmaximum zwischen 250 und 280 nm mit Gipfel bei 265 nm liegt. Versucht man UV-Strahlung dieses Wellenlängenbereichs mit der Chlorung oder Bromung zu kombinieren, so erhält man keine Addition der Einzelwirkungen, da die im bakteriziden Spektralbereich absorbierenden Halogene bzw. mit diesem im Gleichgewicht befindlichen unterhalogenigen Säuren und deren Salze unter Verlust der Oxidations- bzw. Desinfektionskraft photolysiert werden, zugleich aber die das bakterizide UV absorbierenden Oxidations- bzw. Desinfektionsmittel als gelöste Strahlenfilter die Mikroorganismen vor zu starker UV-Strahleneinwirkung bewahren. Im Gegenteil, man benutzt sogar die UV-Strahlung zur Entfernung von aktivem Chlor aus Wasser (Y.Richard in "Oxidationsverfahren in der Trinkwasseraufbereitung", Vortr.veranst. Engler-Bunte Inst. der Univ.Karlsruhe, 11.-13.9.1978; Herausgeb. W.Kühn, H.Sontheimer, Karlsruhe 1979, S. 94 bis 115, insbes. S. 101). Trotz der bekannten Färbung von elementares Jod enthaltendem Wasser findet sich nun im Absorptionsspektrum von Jod in Wasser eine Absorptionslücke mit maximaler Durchlässigkeit für bakterizide Strahlen zwischen 250 und 280 nm, wobei das Maximum der UV-Durchlässigkeit in der 1 cm-Küvette bei ca. 260 nm liegt und z.B. bei 5.5 ppm Jod in Gegenwart von 11.8 ppm Kaliumjodid etwa 99 % beträgt. Bei 254 nm beträgt die Durchlässigkeit noch 97 %, während sie bei 250 nm bereits auf 93 % abgefallen ist. Die UV-Durchlässigkeit (1 cm-Küvette) einer Lösung von 0.55 ppm Jod in Gegenwart von 1.18 ppm Kaliumjodid liegt oberhalb 99.5 % im Bereich von 252 bis 280 nm. Es ist somit bei Desinfektion mit Jod überraschenderweise möglich, dessen günstige Desinfektionseigenschaften praktisch verlustlos mit der hohen Desinfektionswirkung der UV-Strahlung so zu kombinieren, daß die bekannten Nachteile bei der alleinigen Verwendung von Jod bzw. Jodid + Oxidationsmittel aufgehoben werden. Vor allem kann nun bei geringeren Jod/Jodid-Konzentrationen als bisher erforderlich war gearbeitet werden, wodurch sich der Anteil der Kosten verursachenden, zu irreversiblen Jodverlusten führenden Konkurrenzprozesse der Desinfektion verringert. Hierzu gehören insbesondere auch die mit der Erneuerung des Wassers zwangsläufig verbundenen Verluste.

Aber auch der Einfluß der neben der Desinfektion Jod zehrenden Substitutions- und Additionsreaktionen ist bei niedrigeren Jod/ Jodid-Konzentrationen geringer, wobei die UV-Strahlung noch den zusätzlichen Effekt einer Photolyse und damit Rückgewinnung von Jod bzw. Jodid aus den Substitutions- und Additionsprodukten bieten kann. Schließlich gewinnt das Kombinationsverfahren gegenüber der alleinigen Verwendung von Jod dadurch, daß sich in geschlossenen Systemen keine Resistenzen ausbilden können, und daß dank der niedrigeren Jod/Jodid-Konzentration die Probleme unerwünschter Verfärbungen des Beckenwassers zuverlässig auszuräumen sind.

Bei einer alternativen Betrachtungsweise erscheint das erfindungsgemäße kombinierte Desinfektionsverfahren als die beste Lösung der Kombination von UV-Desinfektion mit einem chemischen Restdesinfektionspotential. Versuche, die UV-Bestrahlung mit der Chlorung oder Bromung zu kombinieren, führen wegen der Photolyse des aktiven Halogens, sowie der Strahlenfilterwirkungen zu insgesamt verminderten Desinfektionsleistungen. Erst die überraschende UV-Durchlässigkeit der Jodlösungen im bakteriziden Spektralbereich läßt eine Kombination zu, in der sich die Eigenschaften beider Desinfektionsverfahren summieren oder potenzieren.

Weiterbildungen und vorteilhafte Ausgestaltungen des erfindungsgemäßen Verfahrens sind in den Unteransprüchen gekennzeichnet.

Das Redoxpotential des Wassers zeigt zu jeder Zeit den Gehalt des aktiven Jods an, das im pH-abhängigen Gleichgewicht mit Unterjodiger Säure vorliegt. Es ist daher vorteilhaft, die Zufuhr des Oxidationsmittels in Abhängigkeit von dem Redoxpotential des Wassers zu steuern. Dabei erfolgt die Zufuhr des Oxidationsmittels zweckmäßig anschließend an die UV-Bestrahlung, um eine Photolyse des Oxidationsmittels zu vermeiden. Insgesamt wird dadurch bei dem erfindungsgemäßen Verfahren die Desinfektion durch das Jod zuverlässiger und die Zugabe des Oxidationsmittels sicherer steuerbar.

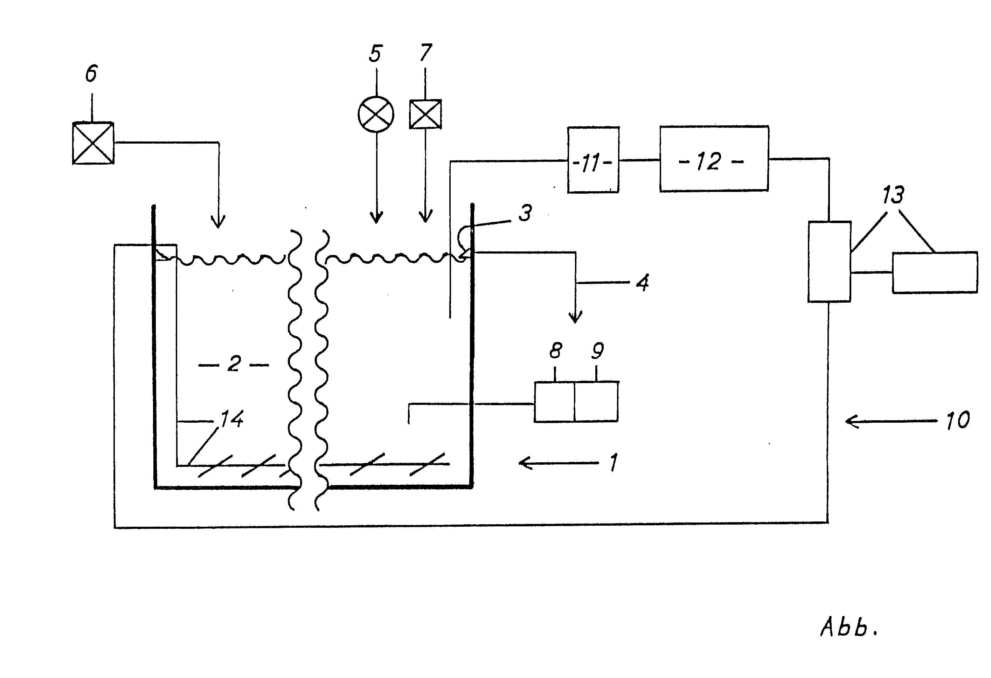

Ein Ausführungsbeispiel für die Anwendung des erfindungsgemäßen Verfahrens in einem geschlossenen System ist schematisch
in der Abbildung dargestellt und wird nachfolgend im einzelnen
erläutert und beschrieben.

Die Abbildung zeigt als Ausführungsbeispiel ein Schwimmbecken 1,
das mit einem Wasservorrat 2 gefüllt ist. Der Rand des Schwimmbeckens 1 ist mit einem sogenannten Skimmer 3 versehen, der
an eine Abflußleitung 4 angeschlossen ist und über den ständig
eine Oberflächenschicht des Wasservorrats 2 abgeschöpft wird.

Das Schwimmbecken 1 weist weiter eine Frischwasserzuführung 5
auf, mittels derer die vorgeschriebene Erneuerung des Wasservorrats 2 bewirkt wird. Das Jodid wird dem Wasservorrat 2 über
eine Dosiervorrichtung 6 zugeführt, wobei Kaliumjodid oder
ein Jodid mit entsprechenden Eigenschaften in Substanz oder
in Lösung zudosiert wird. Die Dosiervorrichtung 6 kann auch
mit der Frischwasserzuführung 5 verbunden sein. Eine weitere
Dosiervorrichtung 7 dient der Zufuhr eines Flockungsmittels.
Weiter sind eine pH-Meßeinrichtung 8 und eine Meßeinrichtung 9
zur Messung des Redoxpotentials des Wasservorrats 2 vorgesehen.

An das Schwimmbecken 1 schließt sich ein Umwälzkreis 10 an, in
dem eine (nicht gezeigte) Fördervorrichtung und in Strömungsrichtung hintereinander eine Filteranlage 11 mit einer nicht
dargestellten Rückspüleinrichtung, eine UV-Bestrahlungseinrichtung 12 und eine Zufuhrvorrichtung 13 für das Oxidationsmittel angeordnet sind und der in einer am Grunde des Schwimmbeckens 1 verlaufenden Verteilereinrichtung 14 endet.

Die UV-Bestrahlungseinrichtung 12 besteht aus einer Strahlungsquelle mit einer Durchflußkammer, in der durch eine hinreichend
hohe Durchflußgeschwindigkeit und gegebenenfalls besondere Strömungsleiteinrichtungen Überbestrahlungen vermieden werden.
Einkammer-Photoreaktoren geeigneter Bauart sind im Handel erhältlich (Vortrag des Anmelders am 15.12.1978, Concept-Seminar
"Good Manufacturing (GMP) in der Kosmetika-Industrie",
Heidelberg; abgedruckt in Parfümerie und Kosmetik), mit Vorteil
sind dafür aber auch Mehrkammer-Photoreaktoren (DE-OS 27 35 550)
einsetzbar.

Die Zufuhrvorrichtung 13 ist nach Art und Ausbildung von dem
jeweils verwendeten Oxidationsmittel abhängig und wird nach
Maßgabe des Redoxpotentials des Wasservorrats 2 eingestellt
bzw. gesteuert. Solche Steuerungen bestehen aus kommerziellen
Komponenten, die in bekannter Weise an die jeweiligen Spezifikationen angepaßt werden, so daß die Zufuhr des Oxidationsmittels nach einem Sollwert des Redoxpotentials ausgerichtet
wird, wobei gegebenenfalls die Einwirkung der UV-Strahlung in
der UV-Bestrahlungseinrichtung 12 zu berücksichtigen ist.

Geeignet sind alle Oxidationsmittel hinreichend hohen Oxidationspotentials, die in den erforderlichen Konzentrationen
im Schwimmbadwasser toxikologisch unbedenklich sind und deren
UV-Absorption den erwünschten UV-Wirkungen nicht im Wege steht.
In Frage kommen z.B. Chlor, Unterchlorige Säure, Natrium- oder
Calciumhypochlorit oder geeignete Chlordonatoren wie N-Chlorverbindungen von der Art des N-Chloramins, des N.N'-Dichlor-
dimethylhydantoins, des Chloramin-T etc., auch Brom und Bromverbindungen wie 1.3-Dibrom-5.5-dimethylhydantoin etc. Für die
Herstellung von Natriumhypochlorit sind Elektrolysezellen für
Kochsalzlösungen geeignet, die im Handel erhältlich sind
(Elcozid; Four-T Engineering Limited, North Dock, Llanelli
SA 15 2LF, Dyfeld, Vereinigtes Königreich). Ferner können als
Oxidationsmittel dienen zahlreiche anorganische wie organische
Derivate des Wasserstoffperoxids, zu denen auch Ozon und Ozonide,
Persäuren und deren Salze zu zählen sind. Von den Peroxiden
seien nur genannt das Natriumperborat-tetrahydrat, das Natriumboratperhydrat, ferner Natriumperoxid, Zinkperoxid und Calciumperoxid oder auch Wasserstoffperoxid für sich allein sowie
Natrium- und Kaliumpersulfat oder auch das als Oxon bekannte
Kaliumsalz der Monosulfopersäure. Von organischen Peroxiden
seien erwähnt Acetopersäure, auch Isopropanol-hydroperoxid
oder Terpinolenendoperoxid-hydroperoxid, die durch photosensibilisierte Reaktionen in Verbindung mit der UV-Bestrahlungseinrichtung 12 kontinuierlich hergestellt und zudosiert
werden können. Auch das von ozonbildenden, bakteriziden Lampen
erhältliche Ozon kann dazu benutzt werden, allerdings wird man
dabei auf die Zugabe eines weiteren Reoxidationsmittels in
keinem Falle ganz verzichten können.

In einem einfachen praktischen Beispiel hat das Schwimmbecken 1
einen Rauminhalt von 112 m³; dieser Wasservorrat wird durch den
Umwälzkreis 10 mit Filteranlage 11 in 2 bis 3 Stunden einmal
umgewälzt. Die mittlere tägliche Belastung liegt bei 200 bis
250 Badenden. Über die Frischwasserzuführung 5 werden 25 m³
pro Tag durch Frischwasser ersetzt. Dem Wasservorrat werden
zunächst 160 bis 180 g Kaliumjodid entsprechend einer Konzentration von 1.4 bis 1.6 ppm zugesetzt und dann entsprechend
der Frischwasserzuführung weitere 35 bis 40 g Kaliumjodid pro
Tag zugeführt. Die wiederholte Oxidation von Jodid zu Jod erfolgt durch Zugabe von Calciumhypochlorit, wobei mit Beginn
des Badebetriebs eine Konzentration von 0.4 ppm Jod eingestellt
wird. Im weiteren Verlauf des Badebetriebs werden je nach der
durch den Abfall des Redoxpotentials gemessenen Belastung
weitere Mengen Calciumhypochlorit zugegeben. Das Kaliumjodid
und das Calciumhypochlorit werden dabei dem Wasservorrat 2
unmittelbar in fester Form zugesetzt. Die Messung des pH-Wertes
und des Redoxpotentials erfolgt in Stichproben mit einer
batteriebetriebenen oder ständig mit einer an das Netz angeschlossenen Meßeinrichtung 8, 9 (z.B. Gerät TM6 bzw. Hydromatic
Pool Controller der Fa. Conducta Gesellschaft für Meß- und
Regeltechnik mbH & Co., Stuttgart). Als UV-Bestrahlungseinrichtung 12 dient beispielsweise eine Wedeco E/30-2 Anlage
(Wedeco Gesellschaft für Entkeimungsanlagen, Düsseldorf-Herford),
die 19 Quarz-Quecksilber-Niederdruckstrahler mit einer Brutto-
Leistungsaufnahme von jeweils 35 W enthält.

In einem weiteren praktischen Beispiel gehört das Schwimmbecken 1 zu einer öffentlichen Badeanstalt; es enthält einen
Wasservorrat 2 von 450 m$^3$, und die mittlere tägliche Belastung
liegt bei 1000 Badenden zwischen 9 und 19 Uhr. Der Wasservorrat 2
wird durch den Umwälzkreis 10 mit Filteranlage 11 in 2.5 Stunden
einmal umgewälzt. Über die Frischwasserzuführung 5 werden 30 m$^3$
pro Tag durch Frischwasser ersetzt. Dem Wasservorrat 2 werden
über die Dosiervorrichtung 6 zunächst 0.7 kg Kaliumjodid entsprechend einer Konzentration von ca. 1.5 ppm und dann unter
Berücksichtigung der Frischwasserzuführung pro Tag weitere
0.1 kg Kaliumjodid zugeführt. Falls die Gesamtkonzentration
an Jod im Wasservorrat 2 über 2.5 ppm ansteigt, wird die tägliche Zugabe von Kaliumjodid soweit reduziert, daß eine stationäre Gesamtkonzentration von 1.5 bis 1.8 ppm Jod eingehalten
wird. Die wiederholte Oxidation von Jodid zu Jod erfolgt durch
die Zufuhr von Hypochloritlauge; die Zufuhrvorrichtung 13 ist
dazu mit einer Elektrolysenzelle (z.B. Elcozid, s.o.) versehen,
die mit Kochsalzlösung beschickt und durch einen auf das Redoxpotential des Wasservorrats 2 ansprechenden Regler (z.B. Hydromatic Pool Controller, s.o.) gesteuert wird. Als UV-Bestrahlungseinrichtung 12 dient eine für einen Durchsatz von
200 m$^3$ pro Stunde ausgelegte Anlage (Wedeco ME/100, 36 Quarz-
Quecksilber-Niederdruckstrahler mit einer Brutto-Leistungsaufnahme von jeweils 35 W; zwei parallel geschaltete Hanovia-
Durchflußreaktoren mit 2.5 kW Quecksilberhochdrucklampen,
Hanovia Lamp Ltd., Slough, Berkshire, Vereinigtes Königreich;
UA-013 Anlage, BBC, Mannheim).

0027278

In den vorstehend beschriebenen Beispielen wird der pH-Wert des Wasservorrats 2 bevorzugt im Bereich von 7.2 bis 8 gehalten. Dies kann dadurch geschehen, daß Soda oder Natriumbicarbonat (1 kg/100 m$^3$) bei niedrigeren und Salzsäure (1 kg/100 m$^3$) bei höheren pH-Werten portionsweise oder durch eine von einem pH-abhängigen Regler gesteuerte Dosiervorrichtung zugegeben werden.

Der Verbrauch an Oxidationsmittel kann nicht genau angegeben werden, weil er von der Temperatur, von der Art des Wassers und vom Umfang der Belastung abhängt. Es kann aber davon ausgegangen werden, daß im Vergleich zur Wasserdesinfektion durch Chlorung der Verbrauch an Oxidationsmittel, bezogen auf Chlor, in dem hier beschriebenen Verfahren nur 10 bis 15 % der im Chlorungsverfahren benötigten Menge Chlor beträgt.

Bei geringeren Belastungen ist es auch möglich, die UV-Bestrahlungseinrichtung 12 kurzzeitig während der Nacht abzuschalten; sie wird dann eine gewisse Zeit vor Beginn des Badebetriebs wieder eingeschaltet.

Als Beispiel für die Anwendung des vorstehend beschriebenen Verfahrens in einem offenen System dient die Trinkwasserversorgungsanlage eines Schiffes, die zwei parallel geschaltete Durchflußphotoreaktoren mit einer maximalen Durchflußleistung von 5 m$^3$ pro Stunde für Wasser mit einer Durchlässigkeit von $\geqslant$ 0.8 (1 cm-Küvette) bei einer Wellenlänge von 254 nm enthält, von denen einer als Reserve dient und in Betrieb genommen wird, wenn der andere ausfällt (z.B. Wedeco E/10 mit Außenbestrahlung, 6 Quarz-Quecksilber-Niederdruckstrahler in individuellen Reflektoren, Brutto-Leistungsaufnahme jeweils 35 W). Vor der Abgabe in die Versorgungsleitung wird das Wasser, dessen bakteriologische Eigenschaften den an Trinkwasser gestellten Anforderungen genügen, zur Sicherheit mit einer Lösung von Jod in Wasser versetzt, die genau auf eine Endkonzentration

0027278

von 0.2 ppm Jod hinzudosiert wird. Dazu können bekannte Einrichtungen zur Herstellung und Dosierung gesättigter Lösungen von Jod in Wasser verwendet werden (A.P.Black et al., l.c. 1965; G.C.White, l.c., S. 693 bis 695); durch exakte Einhaltung einer vorgegebenen Lösungstemperatur ist dabei eine sehr genaue Einstellung der Jodkonzentration in der gesättigten Lösung möglich. Wegen der relativ geringen Jodzehrung ist das aktive Jod im gesamten Trinkwasserleitungssystem präsent.

Es ist auch möglich, das Trinkwasser vor dem Eintritt in die UV-Bestrahlungseinrichtung auf die angegebene Jodkonzentration einzustellen. Dazu ist keine Erhöhung der Jodkonzentration erforderlich.

Selbstverständlich kann auch bei höheren als den bisher genannten Jodkonzentrationen gearbeitet werden, soweit dies aus besonderen Gründen erforderlich ist.

Das elementare Jod kann auch in Form von Jodtinktur oder der Lugolschen Lösung, sowie in Form der sogenannten Jodophore oder ähnlicher löslicher Jodpräparate zugegeben werden (K.H.Wallhäuszer; Sterilisation, Desinfektion, Konservierung; Georg Thieme Verlag 1978, S. 346). Als Jodide sind wasserlösliche, in wässriger Lösung Jodidionen bildende Verbindungen geeignet, wie sie im Handel oder durch übliche chemische Reaktionen erhältlich sind, sofern keine anderweitigen Bedenken gegen ihre Anwendung bestehen. Als Oxidationsmittel für das Jodid kommt auch Jodat in Betracht, das mit Jodid und auch mit Hypojodit bzw. Unterjodiger Säure in einem pH-abhängigen Gleichgewicht der Disproportionierung steht.

Selbstverständlich ist das vorstehend beschriebene Verfahren nicht nur zur Desinfektion von Süßwasser geeignet, sondern auch für Salzwasser, soweit es dessen UV-Durchlässigkeit erlaubt, z.B. auch für Meerwasser. Im Fall der Behandlung von Salzlösungen hinreichender Leitfähigkeit, z.B. auch bei Meerwasser, kann die Freisetzung des Jods aus dem gelösten Jodid auch in einer Durchlaufelektrolysenzelle vorgenommen werden, die zweckmäßigerweise im Umwälzkreislauf 10, gegebenenfalls in Strömungsrichtung hinter der Filteranlage 11, angeordnet wird. Die Durchlaufelektrolysenzelle kann dabei nach Maßgabe des Redoxpotentials des Wasservorrats 2 gesteuert werden.

Patentansprüche

1. Verfahren zur Desinfektion von Wasser mit elementarem Jod, bei dem das Wasser mit Jod versetzt wird, dadurch gekennzeichnet, daß das Wasser mit einer wirksamen Dosis von UV-Strahlung im Wellenlängenbereich von 250 bis 320 nm bestrahlt wird.

2. Verfahren zur Desinfektion eines Wasservorrats, der wenigstens teilweise, gegebenenfalls unter Zwischenschaltung einer Filteranlage, umgewälzt wird, mit elementarem Jod, bei dem der Wasservorrat mit Jodid in der wirksamen Menge, die mindestens zur Erzeugung der zur Desinfektion notwendigen Konzentration an elementarem Jod (in mg/l = ppm) ausreicht, und mit einem Oxidationsmittel in einer Menge versetzt wird, die mindestens zur Erzeugung einer wirksamen Konzentration an elementarem Jod ausreicht, und bei welchem Verfahren das Jodid nach Maßgabe seines Verlustes und das Oxidationsmittel nach Maßgabe seines Verbrauchs ergänzt werden, sowie der pH-Wert des Wasservorrats im Bereich zwischen 6 bis 8 gehalten wird, dadurch gekennzeichnet, daß das Wasser nach Zugabe der nötigen Menge an Jodid im Umwälzkreislauf, gegebenenfalls nach Passieren der Filteranlage, mit einer wirksamen Dosis von UV-Strahlung im Wellenlängenbereich von 250 bis 320 nm bestrahlt wird und das Wasser anschließend an die UV-Bestrahlung mit dem Oxidationsmittel versetzt wird.

3. Verfahren nach Anspruch 1 oder 2, dadurch gekennzeichnet, daß das Wasser mit UV-Strahlung überwiegend im Wellenlängenbereich von 250 bis 280 nm bestrahlt wird.

4. Verfahren nach einem der Ansprüche 1 bis 3, dadurch gekennzeichnet, daß das Redoxpotential des Wassers gemessen und durch Zugabe von Oxidationsmittel in einem Sollwertbereich gehalten wird.

5. Verfahren nach Anspruch 4, dadurch gekennzeichnet, daß das Oxidationsmittel eine wässrige Hypochloritlösung ist, die in einer Elektrolysenzelle aus einer Natriumchloridlösung nach Maßgabe des Redoxpotentials des Wassers erzeugt und diesem hinzudosiert wird.

6. Verfahren nach Anspruch 4, dadurch gekennzeichnet, daß das Oxidationsmittel eine N-Chlor- oder N-Brom-Verbindung ist, die durch eine nach Maßgabe des Redoxpotentials des Wassers steuerbare Dosiervorrichtung zugegeben wird.

7. Verfahren nach Anspruch 4, dadurch gekennzeichnet, daß das Oxidationsmittel eine Peroxyverbindung ist, die durch eine nach Maßgabe des Redoxpotentials des Wassers steuerbare Dosiervorrichtung zugegeben wird.

8. Verfahren nach einem der Ansprüche 2 bis 7, dadurch gekennzeichnet, daß das umgewälzte Wasser über eine Verteilereinrichtung in den Wasservorrat zurückgeführt wird.

9. Verfahren nach einem der Ansprüche 2 bis 8, dadurch gekennzeichnet, daß dem zugeführten Frischwasser Jodid in der jeweils zur Ergänzung des Verlustes erforderlichen Menge zugesetzt wird.

Europäisches
Patentamt

**EUROPÄISCHER RECHERCHENBERICHT**

| Kategorie | EINSCHLÄGIGE DOKUMENTE | | KLASSIFIKATION DER ANMELDUNG (Int Cl ) |
|---|---|---|---|
| | Kennzeichnung des Dokuments mit Angabe, soweit erforderlich, der maßgeblichen Teile | betrifft Anspruch | |
| X | US - A - 3 649 493 (A. MEINERS et al.)  <br> * ganzes Dokument * | 1,3 | C 02 F 1/76 <br> 1/32 |
| X | US - A - 3 232 869 (A.J. GARD)  <br> * ganzes Dokument * | 1-9 | |

RECHERCHIERTE SACHGEBIETE (Int. Cl.³)

C 02 F 1/76
1/32

KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 15-12-1980 | V. AKOLEYEN |

EPA form 1503.1  06.78